# EUROPÄISCHE PATENTANMELDUNG

(11) **EP 2 042 866 A1**
(43) Veröffentlichungstag der Anmeldung: **01.04.2009**
(21) Anmeldenummer: 07117483.3
(22) Anmeldetag: 28.09.2007
(51) Int. Cl.: G01N 33/497, A61B 5/083

(54) **Atemluft-Analyse**

(71) Anmelder: Ionimed Analytik GmbH, 6020 Innsbruck (AT)
(72) Erfinder: Titzmann, Thorsten, 6179 Ranggen (AT); Kohl, Ingrid, 6063 Rum (AT); Beauchamp, Jonathan, 85354 Freising (AT)
(74) Vertreter: Vossius & Partner

(57) **Zusammenfassung**

Eine Atemluft-Probenahmevorrichtung mit einem zu beiden Enden offenen Speicherrohr, wobei Atemluft durch eines der offenen Enden eingeblasen werden kann, und einem Transportrohr, das das Speicherrohr mit einem Messgerät zur Analyse der Atemluft verbindet. Die Probenahmevorrichtung ist derart dimensioniert, dass der alveolare Anteil der Ausatemluft dem Messgerät möglichst unverändert zugeführt werden kann. Weiterhin wird ein Echtzeit-Atemluft-Probenahmesystem und ein Verfahren zur Echtzeit-Analyse von Atemluft unter Verwendung des Probenahmesystems bereitgestellt.

## Beschreibung

Die Erfindung betrifft eine Vorrichtung zum Aufnehmen der Atemluft zur anschliessenden Analyse, sowie ein Verfahren zur Analyse der Atemluft, insbesondere zum quantitativen Nachweis von Stoffen in der Atemluft.

Die Atemluftanalyse hat großes Potenzial als gering invasive Methode zur medizinischen Diagnostik, zum Therapiemonitoring und als Methode für die Quantifizierung einer Exposition mit bestimmten Stoffen (Umweltgifte, Arbeitsplatzbelastungen, etc.). Die Hauptkomponenten der Atemluft sind Stickstoff, Sauerstoff, Kohlendioxid, Wasserdampf und Inertgase. Ein kleiner Anteil der Ausatemluft besteht aus flüchtigen anorganischen und organischen Stoffen (VOC, volatile organic compound). Man kennt mehr als 3400 VOCs in der Ausatemluft normaler gesunder Menschen. Diese Stoffe werden entweder im Körper durch Stoffwechselvorgänge gebildet (endogene Genese) oder aus der Umwelt in den Körper aufgenommen (exogen) und über die Atemluft wieder ausgeschieden.

Man findet heute nur wenige Atemluftanalysemethoden in der klinischen Praxis. Die Ursache liegt in der Komplexität der Zusammenhänge zwischen Atemluftbestandteilen und den physiologischen Vorgängen, die diese Stoffe produzieren, und in methodischen Schwierigkeiten der qualitativen und quantitativen Messung von Spurenstoffen in der Atemluft.

Ziel der Atemluftanalyse ist es, Atemluft-Biomarker für bestimmte Krankheiten zu bestimmen. In der klinischen Praxis ist beispielsweise die Wasserstoffmessung (H₂) in der Atemluft zur Diagnose von Zuckerintoleranz und Zuckermalabsorption anerkannt. Weitere nicht physikalische Atemluftanalysemethoden in der klinischen Praxis sind die NO-Messung zur Diagnose von inflammatorischen Pathologien in den Atemwegen und zur Kontrolle des Therapieerfolges oder der ¹³C-Harnstoff Atemtest zur Diagnose einer Infektion mit Helicobacter Pylori. Nur NO wird durch einen pathologischen Vorgang im Körper vermehrt gebildet. Beim H₂-Atemtest und ¹³C-Harnstoff-Test werden Vorgängerstoffe verabreicht und die Metaboliten dieser Stoffe (Wasserstoffbildung oder ¹³CO₂) in der Atemluft gemessen.

Einen Überblick über Atemluftanalyse in der klinischen Diagnose gibt der Überblicksartikel von W. Cao, Y. Dsan, Breath Analysis: Potential for Clinical Diagnosis and Exposure Assessment", Clin. Chem., 52 (2006) 800-811.

Weiterhin ist eine Vielzahl von Atemtests bekannt, die jedoch in der klinischen Praxis noch nicht etabliert sind. Als Beispiele sind zu nennen:
- Messung der CO-Konzentration in der Ausatemluft zur Bestimmung der Lebensdauer der roten Blutkörperchen bei chronischen Krankheiten, wobei die CO-Konzentration in der Atemluft jedoch stark durch Zigarettenrauchen und durch variierende CO-Konzentration in der Einatemluft beeinflusst wird.
- Messung ¹³C markierter Substanzen als Leberfunktionstests durch Messung bekannter Metaboliten der verabreichten Substanzen. Die genauen metabolischen Reaktionswege sind jedoch noch nicht komplett verstanden.
- Messung von Ammoniak in der Ausatemluft als Maß für die Nierenfunktion und Leberfunktion. Sowohl Patienten mit Leberfunktionsstörungen als auch Nierenfunktionsstörungen haben erhöhte Konzentrationen von Ammoniak in der Atemluft. Auch ältere Menschen haben eine höhere Ammoniakkonzentration in der Atemluft. Deswegen ist Ammoniak kein eindeutiger Atemluftbiomarker für die Nierenfunktion (siehe z.B. K. Toda, J. Li, P. K. Dasgupta, "Measurement of Ammonia in Human Breath with a Liquid-Film Conductivity Sensor", Anal. Chem., 78 (2006) 7284-7291 und darin zitierte Veröffentlichungen). Der Zusammenhang zwischen Ammoniak in der Atemluft und Hartstoff und Kreatinin im Blut wurde etabliert (siehe z.B. M. Phillips, "Breath Tests in Medizine", Sci. Am. 267 (1992) 74-79 oder A. Manolis, "The Diagnostic Potentional of Breath Analysis", Clin. Chem. 29 (1983) 5-15). Im Blutserum wird nicht Ammoniak sondern Harnstoff als dessen Metabolit zu diagnostischen Zwecken bestimmt. Dieser Marker ist jedoch wenig spezifisch. Ammoniakmessungen in der Atemluft werden vorgeschlagen als nicht invasive Entscheidungshilfe für den Dialysefortschritt.
- Messung von Azeton in der Atemluft zur Bestimmung der Ketonämie bei insulinpflichtigen Diabetikern. Atemluft-Azeton wird von vielen metabolischen Vorgängen beeinflusst und ist als Marker wenig spezifisch.
- Propofol in der Ausatemluft: Propofol ist der Handelsname des intravenösen Narkosemittels 2,6-Diisopropylphenol; die Atemluftkonzentrationsbestimmung dient als nicht-invasive Methode für das Monitoring und die Kontrolle der Narkose. Ein genauer Zusammenhang zwischen Atemluftkonzentration und Plasmakonzentration muss erst charakterisiert werden.
- BMAC (Breath Methylated Alcane Contour), Messung eines Musters von methylierten Alkanen in der Atemluft als Metaboliten des oxidativen Stresses bei verschiedenen Pathologien, wie Lungenkrebs, Brustkrebs, Diabetes, Herztransplantat-Abstoßung. Sensitivität und Spezifität von BMAC für konkrete Krankheiten müssen erst etabliert werden.
- Pentan als Marker für den oxidativen Stress und den damit zusammenhängenden Erkrankungen. Pentan ist zu wenig spezifisch, eine Erhöhung wird bei einer Vielzahl von Krankheitszuständen beobachtet.

Zur Atemluftanalyse können Sensoren verwendet werden, die auf unterschiedlichen physikalischen oder chemischen Prinzipien beruhe, z.B. Sauerstoffsensoren, CO₂ Sensoren, etc., sind sensitiv und spezifisch für bestimmte Stoffe. Eine Kopplung der chromatografischen Trenntechnik Gaschromatografie (GC) mit einem Massenspektrometriedetektor (MS) ist unter der Abkürzung GC-MS bekannt. GC-MS ist die am häufigsten in Forschungsarbeiten zu Atemluftanalyse verwendete Standard-Gasanalysemethode. Nachteile sind relative lange Analysezeiten, daher sind mit dieser Methode keine Echtzeitmessungen (online) möglich. Weiterhin ist für die GC-MS die Probennahne und Probenaufkonzentration nötig. GC-MS ist daher eine umständliche, langsame, aber reproduzierbare und für viele Komponenten sensitive Methode. Sie ist in der Lage. Komponenten in Gemischen aus hunderten Stoffen zu trennen und zu identifizieren (siehe z.B. M. Libardoni, P. T. Stevens, J. Hunter Waite, R. Sacks, "Analysis of human breath samples with multi-bed sorption trap and comprehensive twodimensional gas chromatography (GCxGC)", J. Chrom. B, 842, (2006) 13-21 oder R. Zimmermann, W. Welthagen, "Multidimensionale Analytik komplexer Systeme mit GC und MS", Nachrichten aus der Chemie, 54 (2006) 1115-1119).

In verschiedenen Weiterentwicklungen der GC-MS sind eine verbesserte Trennung und verbesserte Detektion erreicht worden. Eine verbesserte Trennung kann durch multidimensionale GC erreicht werden, bei der mehrere Trennschritte mit unterschiedlichen Trennselektivitäten verwendet werden. Diese Methode kann Gemische mit vielen tausend Verbindungen trennen. Die verbesserte chromatografische Trennung verbessert überdies die massenspektrometrische Selektivität. Für die Chromatografie stehen auch verbesserte Detektoren zur Verfügung, z.B. der GCxGC/TOF-MS Detektor. Der TOF-MS Detektor liefert in kürzester Zeit ein gesamtes Massenspektrum mit hoher Massenauflösung. Schwachpunkt hierbei sind die fehlenden chemometrischen Methoden für die Datenanalyse. Anstatt der in der MS herkömmlichen 70eV Elektronenstoßionisationsmethode können weiterhin milde Ionisationsmethode verwendet werden, z.B. Photoionisations-MS, Feldionisations-MS und chemische Ionisations-MS.

Zur Atemluftanalyse wird auch die chemische Ionisations-MS, ohne Kopplung zu Trennsäulen, verwendet. Verschiedenste Reaktanden für die chemische Ionisierungsreaktion sind möglich. Als Beispiele, die in der Atemluft-analyse bereits zum Einsatz kommen, können die PTR-MS (Protonentransferreaktion-Massenspektrometrie), die eine Protonenübertragungsreaktion zur Ionisation verwendet und eine Ionisationsmethode und hochempfindliche Echtzeitanalysemethode darstellt, und die IMR-MS (Ionen-Molekülreaktion Massenspektrametrie) die Kr⁺, Xe⁺, Hg⁺, NO⁺ oder O⁺₂ in der Ionisierungsreaktion verwendet, genannt werden.

Bei der Probenahme einer Atemluftprobe kann zwischen Offline- und Online-Messungen unterschieden werden Bei einer Offline-Messung wird Atemluft aus Gasspeicherbehältern analysiert. Die Speicherung der Atemluftprobe erfolgt in Probenbehältern aus verschiedenen Materialien. Das Einbringen der Probe in das Speicherbehältnis kann sowohl durch unkontrolliertes Einblasen als auch kontrolliert durch einen Apparat erfolgen. Solch ein Apparat misst z.B. CO₂ um einen ganz bestimmten Anteil der Ausatemluft einzufangen. Ein Beispiel für so einen Probenahmeapparat ist der BCA (breath collection apparatus) von Menssana Research Inc. Ein anderes Beispiel ist eine Apparatur, die in J. K. Schubert, K-H. Spittler, G. Braun, K. Geiger, J. Guttmann, "CO2-controlled sampling of alveolar gas in mechanically ventilated patients", J. Appl. Physiol. 90 (2001) 486-492 beschrieben ist.

Die geeignete Speicherung der Atemluftprobe stellt eine wesentliche Schwierigkeit dar, denn die Komponenten der Atemluft liegen nur in geringsten Konzentrationen vor. Der Analytiker muss Sorge dafür tragen, dass durch die Probenspeicherung die Konzentration der Analyten nicht beeinflusst wird. Wesentliche Probleme sind Diffusion durch das Speichermedium, Wechselwirkung mit der Oberfläche und chemische Reaktion in der Gasphase und an der Oberfläche. In weiterer Folge werden die Proben entweder ohne weitere Manipulation gemessen oder unterlaufen weiteren Probebehandlungsschritten. Die Aufbereitung der Proben erfolgt je nach Erfordernis der gewählten analytischen Methode. Beispielsweise wird die Gasprobe über absorbierende Träger geleitet, die die Analyten speichern und konzentrieren sollen. In Folge werden die Analyten durch verschiedene physikalische Methoden in das Messinstrument übertragen, z.B. durch thermische Desorption im Falle der GC-MS.

Im Fall des Online Sampling wird das Atemgas direkt in das Analysegerät eingebracht, entweder aktiv hineingeblasen oder durch das Gerät eingesaugt, und in Echtzeit analysiert. Diese Variante ist schnell und es entfallen die fehleranfällige Speicherung und Aufbereitung. Allerdings eignet sich dieses Verfahren nur für Analysemethoden, die das Atemgas ohne Vorbehandlung verarbeiten können Beispeisweise bereitet die hohe Wasserdampfkonzentration in der Atemluft Probleme im GC-MS. Eine Vorkonzentrierung der Probe ist bei der Echtzeitanalyse ebenfalls nicht möglich, was erhöhte Ansprüche an die Sensitivität der Analysemethode stellt. Die Messmethode muss schnell sein, um zeitaufgelöst zu analysieren. In Frage kommen z.B. die oben beschriebenen chemischen ionisations-massenspektrometrischen Methoden.

Es ist eine Aufgabe der vorliegenden Erfindung, eine verbesserte Probenahmevorrichtung, die eine Echtzeit-Anayse von Atemluft ermöglicht, und ein Verfahren zur Echtzeit-Analyse der Atemluft zum Nachweis bestimmter Stoffe in der Atemluft bereitzustellen Mit der Probenahmevorrichtung soll ein genauer quantitativer Nachweis von Stoffen in der Atemluft bei geringem Zeitaufwand ermöglicht werden.

Diese Aufgabe wird durch die Merkmale der Anspruche gelöst

Die Erfindung basiert auf der Grundidee, ein zu beiden Enden hin offenes Speicherrohr vorzusehen, wobei die Atemluft in eines dieser Enden eingeblasen wird. Über ein Transportrohr wird Luft vorzugsweise in der Mitte aus dem Speicherrohr abgesaugt. Die Dimensionierung des Speicherrohrs ist dabei für eine Messung des alveolaren Anteils eines Atemzuges optimiert. Vom Speicherrohr wird ständig Probenluft über das Transportrohr in das Messgerät, vorzugsweise ein Protoiienaustauselreaktions-Massenspektrometer befördert, sodass in Echtzeit während eines gesamten Atemzuges gemessen werden kann. Zusätzlich speichert das Speicherrohr den letzten, also den alveolaren Teil der Ausatemluft für mehrere Sekunden, der in Folge langsam in das Messgerät transportiert wird. Somit wird der Atemluftstrom durch die kurze Zwischenspeicherung des letzten Anteils des Atemzuges um mehrere Sekunden verzögert und mehr Zeit steht für die Messung zur Verfügung. Zur Verbesserung des Messergebnisses kann die Probenahmevorrichtung geheizt werden.

Beim Einblasen in das Speicherrohr herrschen turbulente Strömungsbedingungen, wodurch es zu einer effektiven Durchmischung im Rohrinneren kommt. Dies dient einer Gleichgewichtseinstellung zwischen inerter Oberfläche und Gasphase. Somit bildet sich von Beginn des Ausatmens an eine Oberflächenbelegung von zu untersuchenden Stoffen aus.

Nach Beendigung des Einblasens wird die im Speicherrohr verbleibende alveolare Atemluft in einer möglichst langsamen laminaren Strömung Richtung Transportrohr abtransportiert, was einer kurzzeitigen Zwischenspeicherung gleichkommt. Durch den Abtransport wird Raumluft ins Speicherrohr nachgesaugt. Durch fehlende Turbulenzen wird der Grenzbereich zwischen Atemluftprobe und Raumluft kaum vermischt (es findet nur eine durch langsame Diffusion verursachte, ineffektive Durchmischung statt), wodurch beinahe das gesamte zwischengespeicherte Gas unverändert analysiert werden kann.

Durch die kurzzeitige Zwischenspeicherung bis zu etwa einer Minute steht für die Analyse eine ausreichende Probenmenge zur Verfügung um valide Daten zu generieren. Durch die Konstruktion des Probenahmesystems zeigen die Messdaten eine starke Signaländerung in einem rechteckigen Verlauf verursacht durch die Änderung von einer alveolaren Gaskonzentration zu einer Umgebungsluftgaskonzentration. Dadurch wird die Unterscheidung zwischen verschiedenen Gasprobenfraktionen sowie die Verarbeitung der Messdaten deutlich vereinfacht.

Die Erfindung stellt weiterhin ein Echtzeit-Atemluft-Probenahmesystem, in dem die erfindungsgemäße Probenahmevorrichtung mit einem Messgerät zur Echtzeitanalyse der Atemluft, vorzugsweise einem Protonentauschreaktions-Massenspektrometer verbunden ist, und ein Verfahren zur Echtzeit-Analyse von Atemluft unter Verwendung dieses Systems bereit.

Die Erfindung wird im Folgenden mit Bezug auf die Figuren näher beschrieben, wobei
Fig. 1 schematisch eine Probenahmevorrichtung gemäß der Erfindung,
Fig. 2 ein Diagramm mit dem Verlauf einer typischen Atemluftmessung,
Fig. 3 ein Diagramm von Atemluft-Messpunkten bei protonierter Molekülmasse von 115 Dalton gegen die zugehörigen Kreatininwerte im Blutserum,
Fig. 4 ein Diagramm von Atemluft-Messpunkten bei protonierter Molekülmasse von 115 Dalton gegen die Tagesharnmenge und
Fig. 5 die chemischen Strukturformeln des Nierenfunktionsparameters Kreatinin (b) und der metabolischen Vorstufe Kreatin (a) zeigt.

Bei der Probenahmevorrichtung gemäß der vorliegenden Erfindung handelt es sich um ein System, das zur Aufnahme, zum Transport und gegebenenfalls zur Erwärmung einer Atemluftprobe vorzugsweise vom Menschen zu einem Gasanalysemessgerät, das in Echtzeit Gasproben analysieren kann, wie z.B. Protonentauschreaktion-Massenspektrometrie (PTR-MS) geeignet ist.

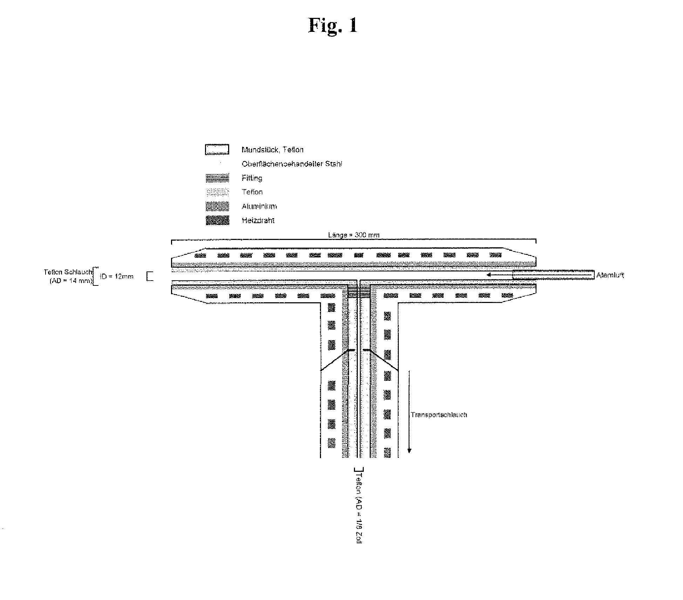

Der Aufbau der Probenahmevorrichtung gemäß einer bevorzugten Ausführungsform der Erfindung ist schematisch in Fig. 1 gezeigt. Die Vorrichtung weist gemäß Fig. 1 ein geheiztes Speicherrohr mit einem Durchmesser von etwa 12 bis 14 mm und einer Länge von etwa 25 bis 35 cm, das aus inerten Materialien wie z.B. Teflon oder oberflächenbehandeltem Stahl oder anderem Material besteht. Weiterhin besteht es aus einem geheizten Transportschlauch, mit einem Außendurchmesser von 1/8 Zoll, der an die Verbindung mit einem Messgerät angepasst ist, einem Innendurchmesser von 0,04 Zoll und einer Länge von 1,5 m, der ebenfalls aus inerten Materialien gefertigt ist. Weiterhin ist ein Mundstück aus Teflon gezeigt, das als Zubehörteil vorgesehen ist, mit einem Durchmesser von etwa 12 mm und einer Länge von etwa 5 cm. Das Mundstück wird von außen etwa 1 cm in das Speicherrohr geschoben und wird nicht beheizt. Das Mundstück ist ein Zubehörteil, das zum Einmalgebrauch bestimmt ist, oder durch Desinfektion für die mehrmalige Verwendung aufbereitet werden kann.

Speicherrohr und Transportrohr sind von einer isolierten Heizmanschette umgeben. Die Temperatur in der Heizummantelung wird durch Temperaturregler konstant gehalten. Dazu kann in der Heizummantelung die Temperatur an einer oder mehr Stellen gemessen werden und entsprechend eines Setpoints gemäß der ermittelten Parameter der PID Funktion durch den Temperaturregler geregelt werden. Die Heizummantelung ist auf eine maximale Temperatur von 100°C ausgelegt. Das Transportrohr ist in der Mitte im rechten Winkel mit dem Speicherrohr verbunden. Das Transportrohr wird am probandenfemen Ende an das Messgerät montiert.

Die Atemluft wird in das System über ein gereinigtes/desinfiziertes Mundstück eingeblasen. Dazu nimmt der Proband das Mundstück in den Mund und bläst die Atemluft vollständig über das Mundstück durch das Speicherrohr. Vom Speicherrohr wird permanent ein Teil der Atemluft mit vorzugsweise 100 ml pro Minute durch das Transportrohr in das Messgerät gesaugt. Der Sog wird durch den herrschenden Unterdruck im Einlassbereich des PTR-MS Messgerätes verursacht und wirkt permanent. Erst wenn das PTR-MS Messgerät ausgeschaltet wird, endet der Sog. Ist das Messgerät nicht dafür ausgestattet, Probenluft durch das Echtzeit-Atemluftprobenahmesystem einzusaugen, wird das Probenahmesystem mit einer entsprechenden Pump Vorrichtung ausgestattet, um den nötigen Volumenstrom zu etablieren.

Nachdem der Proband vollkommen ausgeatmet hat, setzt er das Mundstück ab. Zu diesem Zeitpunkt verweilen im Speicherrohr noch 34 cm³ (bei 1,2 cm Innendurchmesser und 30 cm Länge) bzw. 46 cm³ (bei 1,4 cm Innendurchmesse) alveolare Ausatemluft, die während 20 s bzw. 28 s in das Transportrohr eingesaugt werden. Die Probenluft in den Randbereichen des Speicherrohres vermischt sich durch den Abtransport durch das Transportrohr allmählich mit nachströmender Umgebungsluft bis schließlich nur noch reine Umgebungsluft durch das Speicherrohr ins Transportrohr eingesaugt wird.

Das Mundstück wird nach jeder Atemluftmessung entsorgt oder durch Desinfektion für die Wiederverwendung aufbereitet. Das Mundstück ist ein Zubehörteil.

Bei einem Atemtest mit dem Echtzeit-Atemluftprobenahmesystem gemäß der Erfindung wird der Proband angewiesen, normal ein- und auszuatmen. Das ist wichtig, damit das Gleichgewicht des Gasaustausches in den Alveolen wenig gestört wird. Der Proband wird angewiesen nur ca. jeden 3. Atemzug über das Mundstück auszuatmen. Auch dies dient der Wiederherstellung des Gleichgewichtes an den Gasaustauschflächen der Lunge, da sich die Atemphysiologie bei kontinuierlichem Atmen über ein Mundstück unbewusst ändern könnte. Der Proband wird zusätzlich angewiesen möglichst stetig und vollständig auszuatmen, um die alveolare Ausatemluft im Speicherrohr aufzufangen.

Ausgehend von atemphysiologischen Parametern kann von einem ausgeblasenen Volumen von 500 bis 1000 cm³ bei der Ausatmung durch das Mundstück durch einen Erwachsenen ausgegangen werden. Die Zeitdauer des Ausatmens beträgt ca. 10 Sekunden. Der Volumenstrom durch das Speicherrohr während des Ausatmens beträgt daher 3000 bis 6000 cm³/min durch das Speicherrohr. Sobald der Proband das Mundstück absetzt, endet der Volumenstrom des Ausatmens. Sofort wird durch den Sog aus dem Transportrohr ein gleichmäßiger Sirom Richtung Mitte des Speicherrohres aufgebaut. Der Gasfluss von ca. 100 cm³/min durch das Transportrohr muss durch das Speicherrohr gleichberechtigt von beiden offenen Seiten zur Mitte nachströmen, wo das Transportrohr angeschlossen ist. Deshalb beträgt in jedem Schenkel des Speicherrohres der Volumenstrom ca. 50 cm³ /min.

**Reynoldszahl:** Eine Abschätzung der Strömungseigenschaften ist durch die Berechnung der Reynoldszahl möglich. Bei kleinen Strömungsgeschwindigkeiten verläuft die Strömung ohne Wirbelbildung und man spricht von einer laminaren Strömung. Die laminare Strömung geht in eine turbulente Strömung mit Wirbelbildung bei einer kritischen Reynoldszahl über. Dieser kritische Wert hängt von den Beschaffenheiten der Rohrwandungen ab und beträgt in glatten Röhren Reₖᵣᵢₜ = 1600. Die Reynoldszahl im Speicherrohr liegt während des Ausatmens bei Werten von größer als 2000, d.h. dass eindeutig turbulente Strömungsbedingungen vorliegen. Nach Beenden der Ausatmung ist die Reynoldszahl sehr klein, nämlich kleiner als etwa 200, so dass von laminaren Strömungsbedingungen ausgegangen werden kann. Im Transportrohr ist sie bei allen berücksichtigten Bedingungen sehr hoch, und daher die Strömung immer als turbulent anzusehen. Aus den Reynoldszahlen schließt man, dass die Strömung im Speicherrohr während des Ausatmens turbulent ist. Nach Beenden des Ausatmens wird die verbleibende Atemluft im Speicherrohr in einer laminaren Strömung abtransportiert.
**Transportmechanismen:** Die Strömungscharakteristik hat einen entscheidenden Einfluss auf die Transportmechanismen in den strömenden Gasen. Durch die Turbulenz während des Ausatmens kommt es zur guten Durchmischung des Probengases. Die Konvektion ist dabei der wesentliche Transportmechanismus der Gasmoleküle. Diffusion findet nur noch in kleinsten Bereichen statt und ist unbedeutend. Durch die Durchmischung findet ebenfalls ein Austausch zwischen Gasphasenmolekülen und Rohrinnenwand statt, wodurch die Innenoberfläche konditioniert wird und sich ein Gleichgewicht zwischen Gasphase und Oberflächenbelegung einstellt. Dies konnte auch durch Messungen belegt werden, die die Kondensation von Wasserdampf an der Rohrinnenwand bei niedrigen Temperaturen und die Lösung von Gasphasenstoffen in den Wasserfilm belegen. Bei höheren Temperaturen verschwindet dieser Effekt. well die Wasserdampfkondensation im Wesentlichen ausbleibt. Wenn nach dem Ausatmen die gespeicherte Luft durch das Transportrohr gezogen wird, herrschen laminare Bedingungen. Bei laminaren Strömungen wird die Diffusion der Gasmoleküle zur Rohrinnenwand zwar nicht durch Turbulenzen gestört, allerdings ist die Diffusion durch die vorliegende Gasdichte bei Atmosphärendruck sehr langsam. Dadurch wird die Einstellung einer Oberflächenbelegung im Gleichgewicht mit der Gasphase etwas verzögert. Dieser Effekt tritt auf, wenn sich beim Abtransport der alveolaren Luft aus dem Speicherrohr die gespeicherte alveolare Luft mit der Umgebungsluft vermischt.

Mehrere Vereinfachungen werden für die Charakterisierung des Probenahmesystems angenommen:
**Temperatur:** Für die Berechnungen wird von isothermen Bedingungen innerhalb des Probenahmesystems ausgegangen. Tatsächlich werden die Schläuche des Probenahmesystem bevorzugt auf eine erhöhte Temperatur geheizt. Die strömende Gasprobe innerhalb des Probenahmesystems muss aber erst auf diese Temperatur erwärmt werden. Deswegen liegt wahrscheinlich ein Temperaturgradient zwischen den geheizten Schläuchen und innerhalb der Gasprobe vor. Dieser Temperaturgradient hängt von den Strömungsbedingungen der Gase im Probenahmesystem und der Wärmeleitfähigkeit der Gase und der Rohre ab.
**Gaszusammensetzung:** In erster Näherung wird für die Berechnung der Reynoldszahl der Luft-Wert für die kinematische Viskosität verwendet. Tatsächlich handelt es sich bei den Gasproben um einerseits Ausatemluft mit einem hohen Kohlendioxidgehalt und Wasserdampfgehalt im Prozentbereich, und andererseits um Umgebungsluft, mit ca. 0,04 % Kohlendioxid und variabler Feuchte, je nach Raumklima und Wetterlage.
**Druck:** Der Druck ist in verschiedenen Teilen der Probenahmevorrichtung unterschiedlich. Im Speicherrohr herrscht in erster Näherung ständig Atmosphärendruck, im Transportrohr herrscht ein Druckgefälle von Atmosphärendruck am Eingang bis ca. 300 mbar im Bereich des PTR-MS Messgerätes. Die Reynoldszahl wird in erster Näherung nur für Normaldruck berechnet Auch die durch das Speicherrohr geblasenen Mengen an Atemluft und die dafür benötigte Zeitdauer variiert zwischen den Probanden. Um diese Unsicherheiten miteinzubeziehen werden in der Tabelle jeweils ein maximaler und ein minimaler Wert für eine bestimmte Kenngröße angegeben bzw. berechnet.

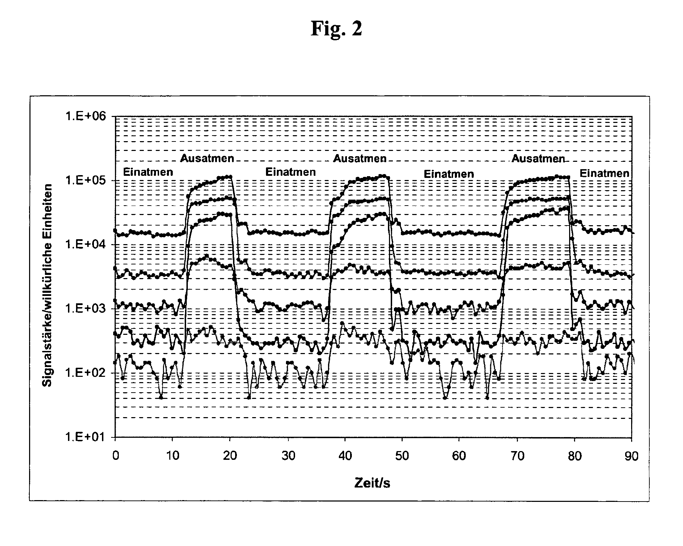

Die Gestaltung der Probenahmevorrichtung ist im wesentlichen auf die Qualität der Messdaten ausgelegt. Die entsprechende Anpassung der Abmessungen dient im Wesentlichen der Erzeugung valider, möglichst durch Rechteckkurven approximierbarer Messdaten. Ein entsprechender Verlauf des Messsignals ist beispielhaft in Fig. 2 gezeigt. Ein möglichst rechteckiges Messsignal reduziert die Anstiegs- und Abfallzeiten der Signale, die für die Messung der alveolaren Luft irrelevant sind. Ferner wird durch ein rechteckiges Signal die automatisierte Erkennung von Atemphasen, sowie Verarbeitung der Messdaten deutlich vereinfacht. Größere Durchmesser des Speicherrohres etwa erzeugen ein höheres Speichervolumen und ermöglichen so längerer Integrationszeiten je Atemzug, allerdings ist die Phase der Rückstellung auf Umgebungsluft, insbesondere wegen verstärkter Diffusion in den Randbereichen, ebenfalls verlängert und die rechte Flanke des Signals verschmiert. Um nach der Ausatmung eine möglichst laminare Strömung mit geringer Durchmischung in den Randbereichen im Speicherohr zu gewährleisten und die Auswirkungen auf die Atemphysiologie möglichst gering zu halten, die durch einen zusätzlichen Atemwiderstand als auch den psychologischen Aspekt entstehen, wurde ein Durchmesser von 12-14 mm für das Mundstück, also gleich dem Innendurchmesser des Speicherrohres gewählt.

Des Weiteren wird sichergestellt, dass genügend Messpunkte von alveolarer Luft zur Verfügung stehen, dass also die Plateauphasen des Signals breit genug sind, um eindeutig die zugehörigen Extremwerte zu erfassen und zu erkennen. Das Volumen des Speicherrohres ist so klein gehalten. dass auch Probanden mit Lungenfunktionsstörungen ein Vielfaches davon an alveolarer Luft einblasen und somit das Rohr gut equilibriert und die Probe sicher alveolarer Herkunft ist

Durch diesen Aufbau ist es möglich den Probanden regelmäßig, aber nicht ununterbrochen in die Probenahmevorrichtung atmen zu lassen und gleichzeitig die zur Verfügung stehende Zeit fast vollständig zur Messung zu nutzen ohne die Atemgasprobe länger als 1 Minute zu speichern oder lange Equilibrierzeiten zwischen Ausatmung und Einatmung in Kauf nehmen zu müssen. Somit wird die Onlinemessung vieler Atemgasbestandteile bei minimalem Zeitaufwand ermöglicht. Die Daten können durch die Probenahmevorrichtung in einer qualitätsgesicherten Weise reproduzierbar aufgenommen werden.

Um die Performance des Echtzeit-Atemluft-Probenahmesystems in einem optimalen Bereich halten zu können, können spezielle Messsequenzen zur Steuerung eingesetzt werden, sowie ein Auswertungsprogramm, welches für dieses Probenahmesystem und die resultierenden Messdaten entwickelt wurde.

Wird das Probenahmesystem zusammen mit einem PTR-MS Messgerät verwendet, so steht eine automatisierte Messgeräte-Steuerungssequenz zur Verfügung. Diese erzeugt Daten in der Art, dass mit einer hierfür konzipierten Softwarelösung vollautomatisch Atemgas- und Umgebungsluftdaten unterschieden werden können. Des Weiteren erfolgt eine automatische Verrechnung von Transmissions- und Normierungsfaktoren, so dass direkt messgerätunabhängige Ergebnisdaten vorliegen. Diese können unmittelbar für statistische Auswertungen und den Vergleich mit anderen Daten herangezogen werden. Die Möglichkeit der vollautomatischen, fehlerfreien Unterscheidung von Atemgasdaten (Ausatmung) und Umgebungsluftdaten (Einatmung) beruht im Wesentlichen auf dem Design des Probenahmesystems zur Erzeugung möglichst rechteckiger Messdatenverläufe. Da das System bauartbedingt stets die gewünschten rechteckigen Messkurven liefert, können auf Basis der Extremwerte der Änderungsraten die Flanken der Atemgasbereiche genau ermittelt werden, dies wäre bei einem weniger steilen Anstieg oder Abfall der gemessenen Konzentrationen nicht möglich.

Eine Anwendung des Echtzeit-Atemluft-Probenahnesystems gemäß der vorliegenden Erfindung ist die Bestimmung der Nierenfunktion durch die Messung von Nrerenfunktionsparametern in der Atemluft.

Zur Überprüfung der Nierenfunktion werden in der klinischen Praxis Kreatinin und Harnstoff im Urin oder im Blutplasma bestimmt. Kreatinin und Harnstoff sind zwei von vielen Stoffen/Toxinen. die sich bei schlechter Nierenfunktion im Blut anreichern. Diesen Zustand bezeichnet man als urämisches Syndrom.

In Deutschland kommt es bei ca. 16000 Patienten jährlich zum totalen Nierenversagen und die Nierenersatztherapie wird nötig. Insgesamt waren in Deutschland im Jahr 2004 60999 Patienten in Dialyse und 21313 Patienten wurden nierentransplantiert. In den USA begannen 2004 102567 Patienten mit einer Nierenersatztherapie (siehe Nierenersatztherapie in Deutschland, Bericht 2004/2005). In Osterreich begannen im Jahre 2005 1211 Menschen mit einer Dialysebehandlung. 2005 wurden in Osterreich insgesamt 7242 Menschen mit Nierenersatztherapie behandelt, 3452 hatten Hämodialyse, 290 Peritonealdialyse und 3500 Menschen lebten mit einem funktionierenden Transplantat (siehe Österreichisches Dialyse- und Transplantationsregister 2005).

Bei der Dialyse werden die Giftstoffe aus dem Blutkreislauf gefiltert, die Körperflüssigkeitsmengen reguliert und die Elektrolyte ausgeglichen. Die Dialyse ist eine lebensrettende Maßnahme und muss mehrmals pro Woche durchgeführt werden. Die Hämodialyse kann die Nierenfunktion nicht vollständig ersetzen. Normale Dialyseroutinen entsprechen etwa 10% der Reinigungskraft der natürlichen Nieren in einer nur intermittierenden Art. Längerfristig kommt es bei Dialysepatienten zu Spätschäden. Je intensiver die Dialyse-Behandlung (Stunden pro Woche), desto länger ist die Überlebenszeit der Patienten. Je älter die Menschen sind, wenn eine Nierenersatztherapie nötig wird, desto kleiner ist die verbleibende Lebenszeit. Man spricht davon, dass die Mortalität von älteren Dialysepatienten ähnlich der Mortalität von Krebserkrankten ist.

Eine zentrale Fragestellung bei der Dialyse ist die Frage der optimalen Dosierung und der Frequenz. Traditionellerweise wird die Effektivität der Dialyse anhand der Reinigung des Blutes vom Harnstoff ermittelt. Die optimale Dialysedauer wird anhand eines kinetischen Modells aus der Konzentration des Harnstoffes im Blut vor der Dialyse und Hämodialyseparametern berechnet. Das Konzept der Dialyseeffektivität beurteilt zusätzlich zu den Labordaten den klinischen Status eines Patienten, wie z.B. Lebensqualität, Blutdruck, Zeichen der Überwässerung, etc. Harnstoff wird als eines von vielen Toxinen, die sich während der dialysefreien Perioden anreichern, quantifiziert.

Zur Beurteilung des Dialysefortschritts ist eine minimal invasive Methode wünschenswert, die in Echtzeit die Konzentrationen einer Vielzahl von Toxinen aufzeichnet. Die Atemluftanalyse kann eine Vielzahl von Markern des urämischen Syndroms in Echtzeit beobachten. Mittels Echtzeit-Atemluftanalyse gemäß der vorliegenden Erfindung durch PTR-MS kann mindestens ein Nierenfunktionsparameter quantifiziert werden, der zur ständigen Bewertung des Dialysefortschritts und zur Festlegung der individuell nötigen verbleibenden Dialysezeit herangezogen werden kann.

Während einer einjährigen Studie führen 110 Patienten kurz vor und nach einer Nierentransplantation jeweils mehrere Atemtests durch. Diese Patienten waren vor der Transplantation in Dialysebehandlung. Nach der Transplantation verbesserte sich die Blutreinigung durch das neue Organ unterschiedlich schnell. Dies wurde durch die Aufzeichnung der Nierenfunktionsparameter erfasst.

Die Bestimmung der Nierenfunktionsparameter erfolgte zu einem anderen Zeitpunkt als die Atemluftmessungen. Für die Auswertung aller Daten wurden die unterschiedlichen Zeitreihen durch folgende Bedingung zusammengeführt: Messwerte aus dem Atemtest gehören zu einem respektiven klinischen Parameter, wenn dieser innerhalb 24 Stunden VOR dem Atemtest gemessen wurde. Durch dieses Vorgehen wurde eine gewisse Ungenauigkeit durch das relativ breite Zeitfenster zur Zusanimenführung der Daten eingeführt. Würde das Zeitfenster kleiner gewählt, verringern sich die verwertbaren Daten Für die Auswertung. Das Ergebnis gewinnt zwar an Genauigkeit, verliert jedoch an Signifikanz.

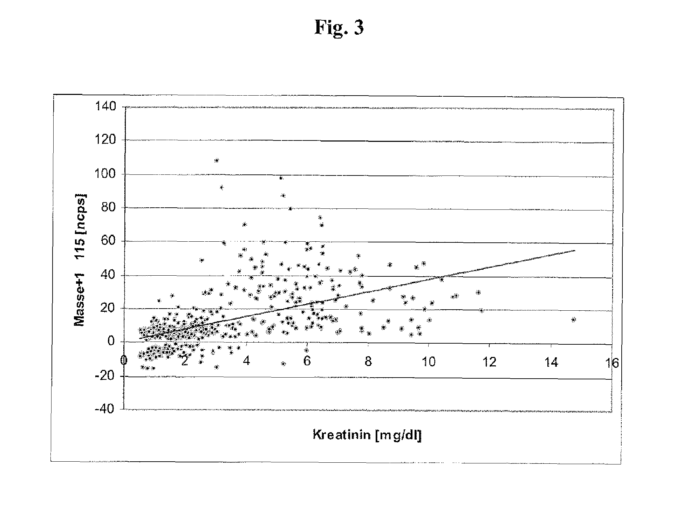

Bei den Atemluftmessungen wurden 41 verschiedene Massen bestimmt. Davon korreliert der Stoff mit der molekularen Masse 114 Dalton eindeutig mit den Nierenfunktionsparametern Kreatinin, Harnstoff (siehe Fig. 3) und der Tagesharnmenge. Der Stoff mit der molekularen Masse 115 Dalton korreliert ebenfalls mit n herkömmlichen Nierenfunktionsparametern und stellt ein Isotop des Stoffes mit der molekularen Masse 114 Dalton dar. In der PTR-MS wird die protonierte Masse gemessen, d.h. molekulare Masse +1, deswegen sind in den Figuren die protonierten Massen angegeben.

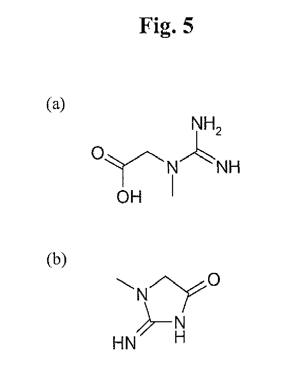

Das PTR-MS Messsignal bei der protonierten Masse 115 Dalton wird durch den Stoff Kreatin verursacht. Die gemessene Massenzahl lässt sich folgendermaßen erklären: Kreatin hat eine molekulare Masse von 131 Dalton. Dieses Molekül fragmentiert bei der Ionisierung im PTR-MS. So wird der Stoff bei einer um eine NH₃-Gruppe geringeren Masse detektiert. NH3 hat eine molekulare Masse von 17 Dalton.
131-17= 114 → protonierte Masse = 115 Dalton.
Kreatin kommt aus der Leber und wird im Muskel als Kreatinphosphat als Engergiequelle für die Muskelaktivität verwendet. Das Abbauprodukt ist Kreatinin, das über die Niere ausgeschieden wird. Die chemischen Strukturformeln von Kreatin und Kreatinin sind in Fig. 5(a) bzw. 5(b) dargestellt.

Die Stoffe mit den Massen 114 und 115 Dalton korrelieren nicht mit dem Auftreten einer Abstoßungsreaktion oder einer Infektion.

In Fig. 3 sind die Atemluft-Messpunkte bei protonierter Masse 115 Dalton gegen die zugehörigen Kreatininwerte im Blutserum aufgetragen. Bei guter Nierenfunktion ist der Kreatinin-Wert im Blutserum gering. Der Normalwert beträgt 0.66 bis 1.25 mg/dl. Bei einer kleinen Kreatinin-Serumskonzentration ist auch das PTR-MS Signal bei Masse 115 Dalton gering und steigt erst mit steigender Kreatininkonzentration, d.h. mit schlechter werdender Nierenfunktion.

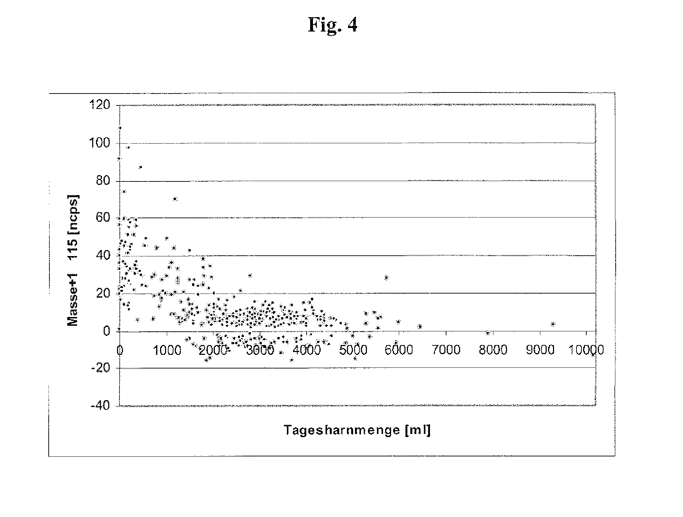

In Fig. 4 ist das PTR-MS Signal bei protonierter Masse 115 Dalton gegen die Tagesharnmenge aufgetragen. Das PTR-MS Signal korreliert auch mit einem weiteren klinischen Parameter für die Nierenfünktion, nämlich der Menge an ausgeschiedenem Urin pro 24 Stunden. Die Harnmenge ist bei niereninsuffizienten Patienten gering bis fehlend. Bei guter Nierenfunktion hängt die Tagesharnmenge von der Flüssigkeitsaufnahme ab. Man erkennt eindeutig einen Zusammenhang zwischen fehlender bzw. geringer Hamproduktion und erhöhtem Signal bei Masse 115 Dalton. Der Knick in der Tendenz der Messpunkte bei einer Tagesharnmenge von ca. 1000 ml markiert den Unterschied zwischen Messungen bei Patienten mit guter Nierenfunktion und Patienten mit schlechter bzw. eingeschränkter Nierenfunktion.

Durch Auswertung der Atemluftdaten aus der oben beschriebenen einjährigen Studie mittels einer statistischen Methode, der Interrelation Miner Methodologie, die zur Datenanalyse ein statistisches Datenmodell für komplizierte Systeme mit einem einfachen Entscheidungsmodell basierend auf Fuzzy Set Theory verbindet, konnte ebenfalls gezeigt werden, dass eine eindeutige Korrelation zwischen den Isotopen mit den Massen 114 und 115 Dalton und der Nierenfunktion besteht. Für die statistische Auswertung wurde die Nierenfunktion dadurch definiert, ob der Patient innerhalb eines Zeitraumes von 48 Stunden Dialyse benötigte oder nicht. Es wurde anhand von Atemluftdaten vorhergesagt, ob der Patient Dialyse hatte oder nicht. Die stärksten Prädiktoren für diese Fragestellung waren die Stoffe mit den molekularen Masse 114 und 115 Dalton.

In der folgenden Tabelle sind die statistischen Kennzahlen von zwei Vorhersagen mit unterschiedlichen Zufallsauswahlen zusammengefasst:

| **Sensitivität** | **Spezifität** | **AUC** | **Dialyse vor Atemtest** |
|---|---|---|---|
| 0,886 | 0,842 | 0,903 | innerhalb 48 Stunden |
| 0,800 | 0,871 | 0,906 | innerhalb 48 Stunden |

Die ähnlichen statistischen Kennzahlen deuten auf eine hohes Maß an Stabilität der Vorhersagen hin. Wie aus der Tabelle ersichtlich ist, konnten sehr hohe Werte für die statistischen Kennzahlen erreicht werden (Zielwerte sind jeweils 1 für eine perfekte Vorhersage, 0,5 würde einen nutzlosen Test anzeigen), was einer Vorhersage hoher Güte entspricht. Dies belegt, dass anhand der in der Atemluft gemessenen Stoffe mit den molekularen Massen 114 und 115 Dalton eindeutig jene Patienten identifiziert werden konnten, die eine Dialysebehandlung benötigten, weil ihre Nierenfunktion schlecht war.

## Patentansprüche

1. Atemluft-Probenahmevorrichtung mit
einem zu beiden Enden offenen Speicherrohr, wobei Atemluft durch eines der offenen Enden eingeblasen werden kann, und
einem Transportrohr, das das Speicherrohr mit einem Messgerät zur Analyse der Atemluft verbindet, wobei die Probenahmevorrichtung derart dimensioniert ist, dass der alveolare Anteil der Ausatemluft dem Messgerät möglichst unverändert zugeführt werden kann.

2. Vorrichtung nach Anspruch 1, wobei das Speicherrohr derart dimensioniert ist, dass der alveolare Anteil der Ausatemluft bis zu etwa einer Minute gespeichert werden kann.

3. Vorrichtung nach Anspruch 1 oder 2, wobei das Speicherrohr einen Innendurchmesser von 12 mm bis 14 mm aufweist.

4. Vorrichtung nach Anspruch 1, 2 oder 3, wobei das Speicherrohr eine Länge von 25 cm bis 35 cm aufweist.

5. Vorrichtung nach einem der vorstehenden Ansprüche, wobei das Transportrohr etwa in der Mitte des Speicherrohrs abzweigt.

6. Vorrichtung nach einem der vorstehenden Ansprüche, wobei das Transportrohr einen Innendurchmesser von 0,2 mm bis 2 mm, vorzugsweise von 1 mm aufweist.

7. Vorrichtung nach einem der vorstehenden Ansprüche, wobei das Transportrohr eine Länge von höchstens 2 m aufweist.

8. Vorrichtung nach einem der vorstehenden Ansprüche, wobei das Transportrohr ein flexible Schlauch ist.

9. Vorrichtung nach einem der vorstehenden Ansprüche, wobei das Speicherrohr und/oder das Transportrohr aus einem inerten Material, beispielsweise Teflon oder oberflächenbehandelter Stahl oder ähnlichen Materialien ist.

10. Vorrichtung nach einem der vorstehenden Ansprüche, wobei die Vorrichtung eine Heizeinrichtung zum Heizen des Speicherrohrs und/oder des Transportrohrs aufweist.

11. Vorrichtung nach Anspruch 10, wobei das Speicherrohr und/oder das Transportrohr von einer isolierten Heizmanschette umgeben ist/sind.

12. Vorrichtung nach Anspruch 10 oder 11, ferner mit mindestens einem Temperatursensor zum Messen und Regeln der Temperatur der Vorrichtung.

13. Vorrichtung nach einem der Ansprüche 10 bis 12, wobei die Vorrichtung auf eine Temperatur von höchstens 250°C und vorzugsweise von 80°C geheizt wird.

14. Vorrichtung nach einem der vorstehenden Ansprüche, ferner mit einem Mundstück, das in einem der beiden offenen Enden des Speicherrohrs angeordnet ist, durch das die Atemluft eingeblasen wird, wobei das Mundstück vorzugsweise aus Teflon oder einem anderen inerten Material gefertigt ist.

15. Vorrichtung nach einem der vorstehenden Ansprüche, ferner mit einer Pumpvorrichtung zum Pumpen von Luft aus dem Speicherrohr durch das Transportrohr in ein Messgerät.

16. Echtzeit-Atemluft-Probenahmesystem mit
einer Atemluft-Probenahmevorrichtung gemäß einem der vorstehenden Ansprüche und
einem Messgerät zur Echtzeitanalyse der Atemluft, wobei das Messgerät vorzugsweise ein Protonenaustauschreaktions-Massenspektrometer ist.

17. System nach Anspruch 16 zum kontinuierlichen qualitativen und/oder quantitativen Nachweis eines Nierenfunktionsparameters in der Ausatemluft.

18. System nach Anspruch 17 zur Bestimmung des Dialysefortschritts auf der Basis des Nachweises des Nierenfunktionsparameters aus der Atemluft.

19. System nach Anspruch 16 oder 17 zur Bestimmung der idealen Dialysedauer bzw. der idealen Dialysefrequenz auf der Basis des Nachweises des Nierenfunktionparameters.

20. Verfahren zur Echtzeit-Analyse von Atemluft unter Verwendung des Systems nach Anspruch 16, mit den Schritten
zyklisches Einblasen von Atemluft in das Speicherrohr,
kontinuierliches Absaugen von Luft aus dem Speicherrohr über das Transportrohr und
Nachweis eines oder mehrerer in der Atemluft enthaltenen Stoffe durch das Messgerät.

21. Verfahren nach Anspruch 20, wobei der bzw. die in der Atemluft enthaltenen Stoffe qualitativ und/oder quantitativ nachgewiesen werden.

22. Verfahren nach Anspruch 20 oder 21, wobei der in der Atemluft nachzuweisende Stoff Kreatin ist beziehungsweise ein anderer Stoff mit der molekularen Masse 114 bzw. 115 Dalton.

23. Verfahren nach Anspruch 20, 21 oder 22, wobei der in der Atemluft nachzuweisende Stoff ein Nierenfunktionsparameter ist.
